# EUROPÄISCHE PATENTSCHRIFT

(11) **EP 3 148 484 B1**
(45) Veröffentlichungstag und Bekanntmachung des Hinweises auf die Patenterteilung: **19.04.2023**
(21) Anmeldenummer: 14728525.8
(22) Anmeldetag: 02.06.2014
(51) Int. Cl.: A61F 2/16, A61B 3/10, G01M 11/02

(54) **VERFAHREN ZUR HERSTELLUNG EINES AUGENIMPLANTATS**
METHOD FOR MANUFACTURING AN EYE IMPLANT
PROCÉDÉ DE FABRICATION D'UN IMPLANT OCULAIRE

(43) Veröffentlichungstag der Anmeldung: 05.04.2017
(73) Patentinhaber: MIRO GmbH, 81241 München (DE)
(72) Erfinder: NEUHANN, Thomas, 80803 München (DE); MÜLLER-LIERHEIM, Wolfgang G.K., 81243 München (DE)
(74) Vertreter: Reichert & Lindner Partnerschaft Patentanwälte
(86) Internationale Anmeldenummer: PCT/EP2014/061391
(87) Internationale Veröffentlichungsnummer: WO 2015/185099

(56) Entgegenhaltungen:
- EP-A1- 1 424 049
- EP-A1- 2 365 379
- WO-A2-2006/060477

## Beschreibung

Die Erfindung betrifft ein Verfahren zur Herstellung eines Augenimplantats nach dem Oberbegriff des Anspruchs 1.

Am optischen System des Auges sind in erster Linie beteiligt die Hornhaut (Cornea) als Eintrittslinse, die Regenbogenhaut (Iris) als Ringblende variablen Durchmessers (Pupille) und die Linse des Auges. Die Blickrichtung des Auges wird durch sechs Muskeln bestimmt. Das Auge kann sein bildgebendes System der Helligkeit der Umgebung anpassen, indem es u.a. den Durchmesser der Pupille verändert. Die Linse ist über Fasern (Zonulafasern) an einem Ringmuskel befestigt. Die Kontraktion dieses Ringmuskels bewirkt eine Erschlaffung der Zonulafasern und dadurch eine stärkere Krümmung der Linsenoberflächen mit der Folge, dass sich die Brennweite der Linse verringert. Dadurch werden anstelle von Bildern in der Ferne solche in geringerer Distanz scharf auf der Netzhaut abgebildet. Dieser Vorgang wird als Akkommodation bezeichnet. Die Fähigkeit der Iris, den Pupillendurchmesser zu verändern sowie die Elastizität und dadurch Akkommodationsfähigkeit der Linse nehmen mit zunehmendem Alter ab.

Die gängigen Verfahren der Augenheilkunde, Optometrie und Augenoptik zur Verbesserung des Sehvermögens haben in erster Linie die Korrektur von Fehlern des optischen Systems des Auges zum Gegenstand. Zu nennen sind hier insbesondere Brillen, Kontaktlinsen, refraktiv-chirurgische Eingriffe zur Veränderung der Hornhautkrümmung, in die Vorderkammer des Auges implantierte sogenannte intraokulare Kontaktlinsen (ICL), nach Entfernung der natürlichen Linse implantierte Intraokularlinsen (IOL), sowie zwischen Iris und natürliche Linse oder Intraokularlinse implantierte Zusatzlinsen.

Sphärische, rotationssymmetrische Linsen und refraktive Hornhauteingriffe dienen der Korrektur der mittleren Brennweite des Auges. Bei sphärischen Linsen wie auch beim Auge werden zentralstrahl-nahe Lichtstrahlen anders abgelenkt als zentralstrahl-ferne Lichtstrahlen. Diese Abweichung wird als sphärische Aberration bezeichnet. Sie lässt sich durch asphärische (rotationsellipsoide) Optikoberflächen korrigieren. In der Praxis kommen diesbezüglich mehrere Korrekturansätze zur Anwendung: Korrektur nur der sphärischen Aberration der verwendeten Linse oder der operativ veränderten Hornhaut, Korrektur der sphärischen Aberration der verwendeten Linse in Kombination mit der aus der Literatur bekannten durchschnittlichen sphärischen Aberration des menschlichen Auges, sowie individuelle Korrektur des Systems aus einem spezifischen Patientenauge und der zum Einsatz kommenden Linse bzw. Hornhautkorrektur.

Das menschliche Auge ist nicht exakt rotationssymmetrisch. Bei signifikanten Abweichungen spricht man von Stabsichtigkeit (Astigmatismus). Zur Korrektur des Astigmatismus kommen torische Linsen (Zylinderlinsen) bzw. eine torische Veränderung der Hornhautform zum Einsatz.

Aber selbst asphärisch-torische Linsen berücksichtigen nicht alle Fehler des optischen Systems des Auges. Die Oberflächen von Hornhaut und Linse weisen unsymmetrische Unregelmäßigkeiten auf. Außerdem befinden sich in der Regel Hornhautscheitel (Apex), Pupillenzentrum, Linsenscheitel und der Punkt schärfsten Sehens der Netzhaut (Fovea) nicht auf einer Achse. Vielmehr orientiert sich das Auge so, dass der Gegenstand, der genau betrachtet werden soll, auf dem Punkt des schärfsten Sehens (Fovea) abgebildet wird, ohne Rücksicht auf damit verbundene zusätzliche optische Fehler des Auges. In der Patentschrift EP 0 954 255 B1 bzw. US 6,215,096 B1 ist ein Verfahren zur Berechnung von Optiken beschrieben, mittels derer eine scharfe Abbildung von Bildern auf der Netzhaut des menschlichen Auges erzielt werden kann.

Die tatsächliche Auflösung der Netzhaut ist bei idealer Abbildung begrenzt durch die Beugung des Lichtes. Zwei räumlich getrennte Punkte sind dann noch unterscheidbar, wenn das Lichtintensitätsmaximum des zweiten Punktes im ersten Beugungsminimum des ersten Punktes liegt. Bei 4 mm Pupillenöffnung und 600 nm Wellenlänge des Lichtes entspricht dies einem Abstand von etwa 4 µm auf der Netzhaut. Der Durchmesser der Rezeptoren an der Stelle schärfsten Sehens in der Netzhaut (Fovea) beträgt etwa 1 bis 3 µm. Dies bedeutet unter anderem, dass selbst bei idealer Bildgebung des menschlichen Auges ein abgebildetes Streifenmuster nie zu Moiré-Effekten führen kann.

Die Akkommodationsfähigkeit des Auges, d.h., die Fähigkeit den Brennpunkt durch Änderung der Linsenkrümmung zu variieren, verringert sich ab der Geburt des Menschen kontinuierlich. Beim normalsichtigen Auge eines 45-Jährigen werden daher in der Regel Gegenstände in weniger als 40 cm Abstand nicht mehr scharf auf der Netzhaut abgebildet. Abhilfe schafft entweder eine Lesebrille, eine Mehrstärken-oder Gleitsichtbrille oder eine Bifokalkontaktlinse. Bei Mehrstärken-, Gleitsichtbrillen und Bifokalkontaktlinsen bestimmt die Blickrichtung die Brennweite der Sehhilfe. Auch die Entfernung der getrübten Augenlinse (Katarakt) mit anschließender Implantation einer Intraokularlinse führt zum Verlust der Akkommodationsfähigkeit des Auges. Hier kommen vereinzelt, mit allerdings bislang geringem Erfolg, sogenannte akkommodierende Intraokularlinsen zum Einsatz. Außerdem werden in 1 bis 2 Prozent der Fälle bi-bzw. trifokale Intraokularlinsen implantiert, deren Optiken diffraktiv oder refraktiv zwei oder drei Brennweiten aufweisen. Bei diesen Intraokularlinsen sieht der Patient stets scharfe und unscharfe Bilder simultan auf der Netzhaut. Weder für die Nähe noch für die Ferne steht die gesamte Lichtmenge in jedem Fokus zur Verfügung.

Aus der EP 2 365 379 A1 ist eine ophthalmische Linse für ein Auge bekannt, wobei das Auge eine scharfe Sehschärfe und eine Tiefenschärfe hat. Die ophthalmische Linse umfasst eine Optik mit einer Mittelachse und einer um die Mittelachse herum angeordneten klaren Apertur. Die Optik umfasst eine erste Oberfläche und eine gegenüberliegende zweite Oberfläche. Die erste und die zweite Oberfläche sind zusammen so konfiguriert, dass sie zumindest eine gewisse asymmetrische Aberration in das Auge einführen, um die Schärfentiefe zu erweitern, während die Sehschärfe im Fokus beibehalten wird. Darüber hinaus sind Angaben zur Herstellung eines Augenimplantats der EP 2 365 379 A1 nicht zu entnehmen.

Aufgabe der vorliegenden Erfindung ist es, ein Verfahren zur Herstellung eines in das Auge implantierbaren Augenimplantats zu schaffen, welches unter Berücksichtigung der der optischen Abbildung nachfolgenden Signalverarbeitung im Auge zu scharfem Sehen in Ferne und Nähe führt.

Diese Aufgabe wird erfindungsgemäß durch die Merkmale des Patentanspruches 1 gelöst. Die Unteransprüche beinhalten Weiterbildungen der Erfindung.

Das erfindungsgemäß hergestellte Augenimplantat kann als Intraokularlinse, welche einen Ersatz der natürlichen Augenlinse bildet, oder als Ersatz einer bereits implantierten Kunstlinse zum Einsatz kommen. Ferner kann das Augenimplantat zusätzlich zur natürlichen Augenlinse, welche im Auge verbleibt, zur Korrektur von Abbildungsfehlern eingesetzt werden. Ferner kann das Augenimplantat zusätzlich zu einer Intraokularlinse, welche die natürliche Augenlinse ersetzt, implantiert werden.

Das erfindungsgemäß hergestellte Augenimplantat besitzt einen Abbildungsfehler des Auges korrigierenden optischen Implantatbereich, welcher ausgehend von biometrisch ermittelten Daten von vor der Augennetzhaut befindlichen optisch wirksamen Bestandteilen und gegebenenfalls von im Auge verbleibenden oder einer künstlichen Augenlinse und deren axialer Position und/oder durch Wellenfrontmessung ermittelten Daten eingestellt ist für ein monofokales Sehen mit einer Sehschärfe von mindestens 0.7 (70 %), bevorzugt mindestens 0.8 (80 %), innerhalb einer Schärfentiefe von mindestens 2 Dioptrien, bevorzugt mindestens 3 Dioptrien.

Die vorstehend angegeben Sehschärfewerte beziehen sich auf photopisches Sehen (Tagsehen, Zapfensehen) bei physiologischer Pupillenweite, die jedoch mindestens einen Durchmesser von 3 mm hat.

Gegenüber zu erwartenden Fertigungstoleranzen und Abweichungen gegenüber genauer Positionierung im Auge sowie intraoperativ und postoperativ zu erwartender Veränderungen des Auges, insbesondere an der Hornhaut, erweist sich das Augenimplantat vorzugsweise als robust. Vorzugsweise gewährleisten die für eine optimale Abbildung eingestellten Abbildungseigenschaften des optischen Implantatbereichs bei Abweichungen des Implantatsitzes im Auge von der vorausberechneten Position im Bereich eines Rotationswinkels von bis zu 2.5°, bevorzugt bis zu 5°, um die Blickrichtungsachse (z-Achse) und im Bereich von Rotationswinkeln von bis zu 1.5°, bevorzugt bis zu 3°, um die dazu senkrechten Lateralachsen (x-Achse, y-Achse) die angestrebte Sehschärfe. Ebenfalls vorzugsweise gewährleisten die für eine optimale Abbildung eingestellten Abbildungseigenschaften des optischen Implantatbereichs bei Abweichungen des Implantatsitzes im Auge von der vorausberechneten Position im Bereich einer Verschiebung von bis zu ± 0.1 mm, bevorzugt bis zu ± 0.2 mm, in Richtung der Blickrichtungsachse und im Bereich von Verschiebungen von bis zu ± 0.2, mm, bevorzugt bis zu ± 0.4 mm, in Richtung der dazu senkrechten Lateralachsen die angestrebte Sehschärfe. Es ist besonders bevorzugt, dass die für eine optimale Abbildung eingestellten Abbildungseigenschaften des optischen Implantatbereichs bei Abweichungen des Implantatsitzes im Auge von der vorausberechneten Position im gesamten durch die vorstehend spezifizierten Rotations-und Verschiebungsabweichungswerte aufgespannten ,Abweichungsraum' die angestrebte Sehschärfe gewährleisten.

Bei der Schaffung des erfindungsgemäß hergestellten Augenimplantats wird die der optischen Abbildung auf der Netzhaut des Auges nachfolgende Signalverarbeitung im neuronalen Sehsystem berücksichtigt.

Der Vorgang des Sehens setzt sich zusammen aus
a) der Abbildung von Bildern der Umgebung mit Hilfe des optischen Systems des Auges auf die Netzhaut (Retina) des Auges,
b) der Umwandlung optischer Reize, die auf die Rezeptoren der Netzhaut treffen, in neuronale Signale der Ganglienzellen (Nervenzellen) in der Netzhaut,
c) der Weiterleitung des visuellen Signals von den Ganglienzellen durch den Sehnerv (Axone der Ganglienzellen) an und der Verarbeitung der neuronalen Signale in den Seitenhöckern (Corpora geniculata), sowie
   der Weiterleitung der Signale an die und deren Weiterverarbeitung in der primären Sehrinde des Hirns,
d) der Weiterleitung der Signale an die sekundäre Sehrinde und an das Großhirn, sowie deren Analyse in der sekundären Sehrinde und dem Großhirn.

Die Netzhaut (Retina) enthält zwei Arten von Lichtrezeptoren, nämlich ca. 120 Millionen Stäbchen und ca. 6 Millionen Zapfen. Die Stäbchen sind verantwortlich für die Detektion lichtschwacher Signale. Die Zapfen sind verantwortlich für die hohe räumliche Auflösung von Objekten und die Farberkennung. Dazu gibt es drei unterschiedliche Zapfenarten, die sich in der Wellenlänge des Lichtabsorptionsmaximums unterscheiden. Die höchste Dichte der Zapfen ist im Punkt schärfsten Sehens der Retina (Fovea centralis, Sehgrube, Einsenkung mit etwa 1.5 mm Durchmesser im Zentrum der Macula lutea, gelber Fleck). Die höchste Dichte der Stäbchen ist an der Peripherie der Fovea centralis.

Die insgesamt etwa 126 Millionen Lichtrezeptoren senden ihre Signale an nur etwa 1 Million Ganglienzellen (Nervenzellen), deren Nervenfasern (Neuronenfasern, Axone) den Sehnerv (Nervus opticus) bilden. Von den 1 Million Axonen gehen etwa 10 Prozent zum Colliculus superior und dienen der Steuerung von Augenbewegung, Pupillengröße und Akkommodation der Linse, die restlichen 90 Prozent gehen zu den seitlichen Kniehöckern des Thalamus (Corpus geniculatum laterale, CGL) und dienen der visuellen Wahrnehmung.

Nervenzellen (Neuronen) bestehen aus dem Zellkörper (Soma), Neuronenfasern (Axonen) mit Verzweigungen und Verästelungen (Dendriten). Jedes Neuron kann Signale (Nervenimpulse, neuronale Impulse) von vielen vorgeschalteten Neuronen empfangen und an viele nachgeschaltete Neurone weiterleiten. Bei neuronalen Signalen handelt es sich um elektrische Impulse im Bereich von etwa 100 Millivolt auf der Basis chemischer Prozesse. Das Neuron entlädt sich (,feuert') in einem alles-oder-nichts-Prozess. Die Reizamplitude wird umgesetzt in die Häufigkeit und Regelmäßigkeit der Nervenimpulse. Nach jedem Impuls benötigt das Neuron mindestens 1 bis 2 Millisekunden Erholungspause, d.h., die maximale 'Feuerrate' ist 500 bis 800 Impulse pro Sekunde. Auch ohne Reiz feuern die Neurone mit geringer Rate (Spontanrate). Die Reizamplitude ist daher begrenzt durch die maximale Feuerrate und die Spontanrate.

Der Impuls wird vom Zellkörper (Soma) des Neurons zum Axon gesendet. Das Axonende befindet sich in der Nähe der Dendriten oder des Zellkörpers eines nachfolgenden Neurons, aber räumlich getrennt durch einen Spalt (Synapse). Die Übermittlung des Impulses erfolgt durch Ausschüttung einer chemischen Substanz (Neurotransmitter) durch das präsynaptische Neuron. Neurotransmitter lagern sich an die Zellmembran des postsynaptischen Neurons an. Je nach Typ des Neurotransmitters und der postsynaptischen Zellmembran kann der präsynaptische Impuls anregend (Steigerung der Feuerrate) oder hemmend (Verringerung der Feuerrate) auf das postsynaptische Neuron wirken. Jedes Neuron empfängt in der Regel von mehreren anderen Neuronen anregende und hemmende Impulse, die in der Summe die eigene Impulsrate im Vergleich zur Spontanrate steigern, verringern oder unverändert lassen.

Beim Zusammenwirken von Neuronen ist vereinfacht zu unterscheiden zwischen linearer Schaltung, Konvergenzschaltung und Konvergenzschaltung mit seitlicher (lateraler) Hemmung. Bei der linearen Schaltung gibt es genau eine Synapse zwischen einem Rezeptorneuron und dem weiterleitenden Neuron, und das im Rezeptorneuron erzeugte Signal wird nur an dieses eine weiterleitende Neuron weitergegeben. Alle Synapsen sind erregend, das bedeutet eine lineare Weiterleitung des Signals ohne Verarbeitung. Bei der Konvergenzschaltung konvergieren mehrere Rezeptorneuronen mit ihren Axonen auf die nächste und übernächste Ebene, wobei alle Synapsen erregend sind. Dies führt zu einer Steigerung der Detektion schwacher Signale, wobei gleichzeitig z.B. auf der Netzhaut die Ortsauflösung sinkt. Bei der Konvergenzschaltung mit lateraler Hemmung konvergieren die Rezeptorneuronen auf der nächsthöheren Ebene von Neuronen; gleichzeitig sind die Synapsen seitlicher Neurone auf der nächsthöheren Ebene hemmend. Dies führt zu maximaler Erregung, wenn der Reiz eine gewisse räumliche Ausdehnung hat (,ON-Bereich') und Hemmung bei weiterer räumlicher Ausdehnung ('OFF-Bereich'). Bezogen auf die Retina ist die Konvergenzschaltung mit lateraler Hemmung unter anderem die Basis für die bereits auf retinaler Ebene einsetzende ,Bildschärfung'. In der Retina erfolgt die Verschaltung der Rezeptoren (Zapfen und Stäbchen) mit den weiterleitenden Ganglienzellen linear, konvergent und konvergent mit lateraler Hemmung durch die Bipolar-, Horizontalund Amakrinzellen. Die Konvergenzschaltung der Stäbchen ist für die hohe Lichtempfindlichkeit verantwortlich, wobei die Ganglienzellen erst bei gleichzeitiger Erregung durch mehrere Stäbchen 'feuern'. Die Zapfen in der Fovea centralis sind mit den Ganglienzellen linear verschaltet, was zur höheren Ortsauflösung beiträgt. Der Bereich der Netzhaut, auf den ein einzelnes Neuron einer höheren Ebene anspricht, wird als rezeptives Feld dieses Neurons bezeichnet.

Die optische Abbildungsqualität des durchschnittlichen menschlichen Auges ist relativ schlecht. Die Oberflächen von Hornhaut und Linse weisen unsymmetrische Unregelmäßigkeiten auf. Außerdem befinden sich in der Regel Hornhautscheitel (Apex), Pupillenzentrum, Linsenscheitel und der Punkt schärfsten Sehens der Netzhaut (Fovea centralis) nicht auf einer Achse. Dennoch orientiert sich das Auge so, dass der Gegenstand, der genau betrachtet werden soll, auf dem Punkt des schärfsten Sehens (Fovea) abgebildet wird, ohne Rücksicht auf damit verbundene zusätzliche optische Fehler des Auges. Die optische Abbildung eines Punktes erfolgt deshalb im Auge als Fleck unterschiedlicher Größe, je nach der Unvollkommenheit des individuellen optischen Apparates. Dass das Auge dennoch scharfe Bilder wahrnimmt, verdankt es seiner Fähigkeit zur Bildschärfung` (laterale Kontrastverarbeitung), die insbesondere in der Retina beginnt.

Ein scharfer Punkt der Umwelt wird also durch die bildgebende Funktion des Auges auf der Retina als Lichtintensitätsverteilung über eine Vielzahl von Rezeptoren als Fleck abgebildet. Erst die neuronale Bildschärfung führt zur Wahrnehmung eines scharfen Punktes.

Das menschliche Auge weist selbst im hohen Alter noch eine Schärfentiefe von etwa 0.75 Dioptrien auf. Da die natürliche Augenlinse in diesem Alter nicht ackommodiert, ist diese 'Schärfentiefe' vermutlich der oben erläuterten neuronalen Bildschärfungsfähigkeit des Auges zu verdanken.

Bei der Erfindung werden die erläuterten Bildschärfungsfähigkeiten des Auges durch laterale Signalverarbeitung der Netzhaut genutzt, um eine optimale Schärfentiefe mittels eines monofokal abbildenden Augenimplantats zu erzielen, wobei ein scharfes Sehen von Objekten in unterschiedlicher Entfernung erreicht wird.

Das erfindungsgemäße Prinzip besteht dabei darin, dass die optische Abbildung bereits so optimiert wird, dass die laterale Kontrastverarbeitungskapazität des visuellen Systems, zuvörderst der Retina, nun nur noch in minimalem Ausmaß zur Beseitigung der Abbildungsfehler im Fokus benötigt wird - sie steht deshalb in wesentlichem Ausmaß für die Schärfung von durch Defokus verursachter Unschärfe zur Verfügung und kann deshalb zur Erhöhung der natürlichen Schärfentiefe genutzt werden.

Anhand der Figuren wird die Erfindung noch näher erläutert. Es zeigt:
- Fig. 1: eine beispielhafte Darstellung der Sehschärfe, die mit einem Augenimplantat gemäß einer Ausführungsform der Erfindung innerhalb einer bestimmten Schärfentiefe erreicht wird,
- Fig. 2: eine beispielhafte Lichtintensitätsverteilung der von dem Augenimplantat gemäß der Ausführungsform auf der Netzhaut des Auges erzeugten Abbildung, und
- Fig. 3: ein Ausführungsbeispiel der Arbeitsschritte zur Herstellung des Augenimplantats gemäß der Ausführungsform in einem Flussschema.

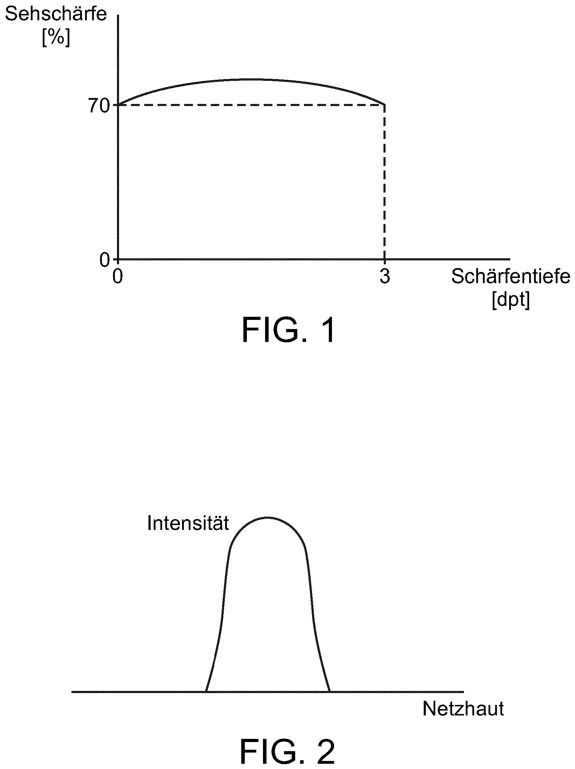

In der Fig. 1 ist die Sehschärfe, die mit einem Augenimplantat gemäß einer Ausführungsform der Erfindung innerhalb einer bestimmten Schärfentiefe, hier von 3 Dioptrien, erreicht wird, dargestellt. Diese Sehschärfe beträgt für die Fernsicht (0 Dioptrien) und für die Nahsicht (3 Dioptrien) 0.7 (70 %). Die Sehschärfe bezeichnet die Fähigkeit des Auges bei optimaler Korrektur durch das Augenimplantat zwei Objektpunkte getrennt wahrzunehmen. Dies ist möglich, wenn in der Fovea centralis der Augennetzhaut zwischen zwei gereizten Zapfen der dazwischen liegende Zapfen nicht mehr als 70 % gereizt wird. Die Rastergröße beträgt hier 2 µm.

In der Fig. 2 ist die Lichtintensitätsverteilung der vom Augenimplantat auf der Netzhaut des Auges erzeugten Abbildung dargestellt. Hierdurch wird eine laterale Signalverarbeitung der Netzhaut verursacht, durch welche eine optimale Schärfentiefe mit einer Sehschärfe von mindestens 0.7 (70 %) für Fernsicht und Nahsicht erreicht wird.

Vor der Implantation wird das Auge biometrisch vermessen (Topographie von Hornhautvorder-und -rückfläche, Achslänge des Augapfels und bei phaken Implantaten und intraokularen Implantaten Position sowie Topographie von Vorder- und -Rückfläche des Implantats) und aus diesen Messwerten in Kombination mit der geplanten Position des Implantats im Auge und dem Brechungsindex des Materials die Oberflächentopographie als Solltopographie des ins Auge zu implantierenden Implantats berechnet. Ferner können, insbesondere für Zusatzlinsen zur Implantation in phake oder pseudophake Augen, durch Wellenfrontmessung der abbildenden Bestandteile die für die Berechnung des optischen Implantatbereiches erforderlichen Daten bestimmt werden.

Zur Ermittlung der Eigenschaften der lichtbrechenden Bestandteile des Auges wird vorzugsweise der vordere Augenabschnitt untersucht. Hierzu eignet sich beispielsweise eine Scheimpflugkamera, mit welcher berührungslos Schnittbilder der vorderen Augenkammer aufgenommen werden können. Diese Aufnahmen ermöglichen eine Analyse der gesamten Hornhaut, der Vorderkammer und der natürlichen Linse. Dabei können geometrische Daten wie Zentralradien, Hornhautasphärizität, verschiedene Krümmungen der Hornhaut, der Kammerwinkel, das Kammervolumen sowie die Kammerhöhe der Vorderkammer und auch Linsentrübungen analysiert werden. Beispielsweise aus EP 1074214 B1 ist eine derartige Analyse des vorderen Augenabschnitts bekannt.

Aus den Analysedaten lässt sich vorteilhafterweise auch die Position des Implantates im Auge vorhersagen.

Aus diesen bei der Analyse gewonnenen Daten sowie den bekannten Brechungsindizes insbesondere der Hornhaut und des Augenkammerwassers wird für das Implantat eine Solltopographie an einer der beiden Implantatoberflächen oder für beide Implantatoberflächen berechnet. Dabei wird auch das für das Implantat zu verwendende Material berücksichtigt. Hierbei kann es sich um kommerziell erhältliche Polymere, wie beispielsweise MMA/HEMA-Copolymere handeln. Ein geeignetes Material ist beispielsweise auch aus WO 2007/062864 bekannt.

Das Implantat kann jedoch aus jedem implantierbaren Material mit optischer Qualität hergestellt werden.

Die Fertigung der Optik des Augenimplantats; insbesondere der individuellen, implantierbaren Linse erfolgt mit Standardverfahren, wobei eine der Optikflächen eine Standardgeometrie (sphärisch, asphärisch oder torisch) aufweisen und durch Drehen, Gießen oder Spritzprägen hergestellt werden kann. Die zweite Optikfläche wird vorzugsweise mit einer für die Herstellung von Freiformflächen geeigneten, programmierbaren Drehbank oder Laserbestrahlung, insbesondere Lasernachbearbeitung erzeugt, wobei Arbeitsschritten der Vorzug gegeben wird, bei denen eine anschließende Politur der Oberfläche entfallen kann. Auch die Herstellung durch Direktgießen mit entsprechend geformten Gießwerkzeugen ist möglich.

Aus der Solltopographie werden Maschinendaten berechnet, welche zur Steuerung der mechanisch spanabhebenden Bearbeitung oder Lasernachbearbeitung der Oberfläche eines Standardrohlings geeignet sind. In Abhängigkeit von diesen Maschinendaten erfolgt dann beispielsweise in einer geeigneten Dreh-oder Fräsmaschine die mechanisch spanabhebende Bearbeitung an der Oberfläche des Standardrohlings. In bevorzugter Weise kommt eine Dreh-und/oder Fräsmaschine zum Einsatz, mit welcher die Bearbeitung der Oberfläche mit einer solchen Präzision erfolgt, dass eine anschließende Politur nicht erforderlich ist. Vorzugsweise kommt hierbei ein Diamantwerkzeug in der Drehmaschine zum Einsatz. Bei dem Standardrohling, aus welchem das Implantat, insbesondere die individuelle Intraokularlinse, durch mechanisch spanabhebende Bearbeitung oder Laserbearbeitung hergestellt wird, handelt es sich vorzugsweise um einen durch Spritzprägen gefertigten Rohling. Auf diese Weise erhält man einen Rohling mit genau vorgegebenen Abmessungen an den Oberflächen, von denen ausgehend die gewünschte Topographie durch die mechanisch spanabhebende Bearbeitung oder Laserbearbeitung erzeugt wird.

Beim Spritzprägen ist es auch möglich die Haptik, welche zur Fixierung des Implantats im Auge dient, an das Implantat anzuformen.

Die Qualitätskontrolle der Topographie der als Freiflächen gestalteten Optiken erfolgt vorzugsweise durch Analyse der gemessenen, insbesondere der reflektierten Wellenfront, wobei die Sollfläche als mathematische Referenz gewählt und Abweichungen der Topographie anhand der Analyse der gemessenen Wellenfront von der erwarteten Wellenfront berechnet werden. Die Wellenfrontmessung erfolgt vorzugsweise bei einer Wellenlänge, bei der das nicht reflektierte Licht vom Implantatmaterial absorbiert wird, um die Überlagerung der reflektierten Wellenfront der Frontfläche der Optik durch Reflexionen von der Rückfläche der Optik zu minimieren.

Die Messung der Topographie der Implantatoberfläche kann beispielsweise mit Hilfe eines Wellenfrontsensors, der als Shack-Hartmann-Sensor ausgebildet ist, erfolgen. Der Shack-Hartmann-Sensor enthält eine Anordnung von Mikrolinsen in deren Brennebene ein ortsauflösender Lichtsensor, welcher beispielsweise als CCD-Sensor ausgebildet sein kann, angeordnet ist. Die gemessene Topographie verursacht Wellenfronten, welche ein Auslenken der Brennpunkte der Mikrolinsenanordnung auf dem ortsauflösenden Lichtsensor bewirken. Hieraus lassen sich Messergebnisse für die hergestellte Topographie auf der Implantat-oberfläche gewinnen.

Die Messung mithilfe des Shack-Hartmann-Sensors kann im Durchlichtverfahren, bei welchem das bei der Messung verwendete Licht durch den optischen Implantatbereich hindurch gestrahlt wird, gemessen werden. Es kann jedoch bevorzugt auch eine reflektierende Messmethode, bei welcher an der Implantatoberfläche reflektiertes Licht vom Shack-Hartmann-Sensor erfasst wird, verwendet werden. Diese Messmethoden sind beispielsweise aus DE 20 2008 004 608 A1 für die Erfassung von Implantatfehlern bekannt.

Auch ein die Oberfläche des optischen Implantatbereichs abtastender Topographiesensor, welcher als Abstandssensor oder Winkelsensor ausgebildet ist, können zur Topographiemessung an der Implantatoberfläche verwendet werden.

Ein derartiger Topographiesensor ist beispielsweise aus WO 2009/124767 bekannt.

Die gemessene Topographie des optischen Implantatbereichs wird mit der Solltopographie verglichen. Hierzu werden die Messergebnisse auf das Format der Solltopographie umgerechnet. Es ist jedoch auch möglich die Solltopographie dem Format der gemessenen Topographie für den Vergleich anzupassen.

Auf der Implantatoberfläche kann an einer der beiden Oberflächen die gewünschte Topographie hergestellt werden. Es ist jedoch auch möglich, an beiden Oberflächen (Vorderseite und Rückseite des Augenimplantats) jeweilige Topographien, die individuell gestaltet sind, zur Korrektur der Fehlsichtigkeit, welche aus den Augenbestandteilen resultiert, herzustellen.

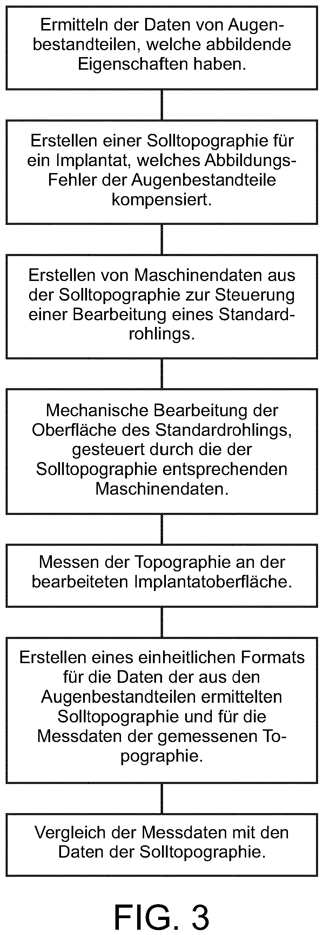

In der beigefügten Figur 3 sind für ein Ausführungsbeispiel die verschiedenen Schritte zur Herstellung des Augenimplantats, insbesondere einer Intraokularlinse, gemäß der Ausführungsform in einem Flussschema dargestellt.

## Patentansprüche

1. Verfahren zur Herstellung eines Augenimplantats, enthaltend:
einen optischen Implatatbereich zum Korrigieren eines Abbildungsfehlers des Auges,
**dadurch gekennzeichnet,**
**dass** eine Oberflächentopographie des optischen Implatatbereichs basierend auf biometrisch bestimmten ermittelten Daten von vor der Augennetzhaut befindlichen optisch wirksamen Bestandteilen und/oder von durch Wellenfrontmessung ermittelten Daten eingestellt wird, um Abbildungsfehler des Auges so zu korrigieren, dass der optische Implantatbereich in einer solchen Weise optimiert wird, dass die laterale Kontrastverabeitungskapazität der Retina nur in einem minimalen Ausmaß zum Korrigieren der Abbildungsfehler im Fokus benötigt wird und daher in einem hohen Ausmaß zum Schärfen des durch Defokus verursachten unscharfen Sehens zur Verfügung steht und die natürliche Schärfentiefe erhöht,
wobei aufgrund der korrigierten Abbildungsfehler an dem optischen Implantatbereich die laterale Signalverarbeitung in der Retina ein optimales monofokales Sehen mit einer Sehschärfe von mindestens 0.7 (70 %) innerhalb einer Schärfentiefe von mindestens 2 Dioptrien erzielt, und
**dass** nach Bestimmung einer Solltopographie für wenigstens eine der beiden Oberflächen des optischen Implantatbereichs, gesteuert durch die bestimmte Solltopographie, wenigstens eine von beiden Oberflächen eines Standardrohlings spanabhebend oder durch Laserstrahlung bearbeitet wird oder durch Direktgießen mittels entsprechend ausgebildeter Gießformen hergestellt wird und dass die so gefertigte Topographie an wenigstens einer Implantatoberfläche gemessen und mit der Solltopographie verglichen wird.

2. Verfahren nach Anspruch 1, **dadurch gekennzeichnet, dass** für die Bestimmung der Solltopographie die optischen Daten von wenigstens zwei der folgenden Augenbestandteile biometrisch gemessen werden: Hornhaut, Vorderkammer, natürliche Linse, Position der natürlichen Linse oder der die natürliche Linse ersetzende bereits implantierten Kunstlinse gegenüber der Hornhaut, Achslänge des Augapfels.

3. Verfahren nach Anspruch 1 oder 2, **dadurch gekennzeichnet, dass** geometrische Daten und/oder Brechungsindizes der Augenbestandteile für die Bestimmung der Solltopographie verwendet werden.

4. Verfahren nach einem der Ansprüche 1 bis 3, **dadurch gekennzeichnet, dass** aus den biometrisch ermittelten Daten des Auges der Sitz der zu implantierenden Intraokularlinse im Auge bestimmt wird.

5. Verfahren nach einem der Ansprüche 1 bis 4, **dadurch gekennzeichnet, dass** die gemessenen Daten der durch mechanisch spanabhebende oder Laser-Bearbeitung hergestellten Topographie an der Linsenoberfläche und/oder die Daten der Solltopographie für den Vergleich in ein einheitliches Format gewandelt werden.

## Claims

1. Method for manufacturing an eye implant, comprising:
an optical implant region for correcting an aberration of the eye,
**characterised**
**in that** a surface topography of the optical implant region is set on the basis of biometrically determined acquired data from optically effective components located in front of the retina and/or from data acquired by wave front measurement, so as to correct aberrations in the eye in such a way that the optical implant region is optimised in such a way that the lateral contrast processing capacity of the retina is required only to a minimal extent for correcting the aberrations in focus, and therefore is highly available for focussing the unfocussed vision due to defocussing and increases the natural depth of focus,
wherein the lateral signal processing in the retina achieving optimal monofocal vision with a visual acuity of at least 0.7 (70 %) within a depth of focus of at least 2 dioptres, as a result of the corrected aberrations at the optical implant region, and
**in that**, after a target topography for at least one of the two surfaces of the optical implant region is determined, at least one of the two surfaces of a standard blank is processed by machining or laser-cutting or is produced by direct casting using appropriately formed casting moulds, in a manner controlled by the particular target topography, and in that the topography thus produced is measured at at least one implant surface and compared with the target topography.

2. Method according to claim 1, **characterised in that**, for determining the target topography, the optical data from at least two of the following eye components are measured biometrically: cornea, anterior chamber, natural lens, position of the natural lens or of the already implanted artificial lens replacing the natural lens with respect to the cornea, axial length of the eyeball.

3. Method according to either claim 1 or 2, **characterised in that** geometric data and/or refractive indices of the eye components are used for determining the target topography.

4. Method according to any of claims 1 to 3, **characterised in that** the seat, in the eye, of the intraocular lenses to be implanted is determined from the biometrically acquired data of the eye.

5. Method according to any of claims 1 to 4, **characterised in that**, for the comparison, the measured data of the topography, manufactured by mechanical machining processing or laser processing, at the lens surface and/or the data of the target topography are converted into a unitary format.

## Revendications

1. Procédé de fabrication d'un implant oculaire comprenant :
une zone d'implant optique pour corriger une aberration de l'œil,
**caractérisé en ce**
**qu'**une topographie de surface de la zone d'implant optique est réglée sur la base de données déterminées biométriquement de composants optiquement actifs situés devant la rétine de l'œil et/ou de données déterminées par mesure du front d'onde, afin de corriger des aberrations de l'œil de telle sorte que la zone d'implant optique soit optimisée de manière à ce que la capacité de traitement de contraste latéral de la rétine ne soit requise que dans une mesure minimale pour corriger les aberrations au foyer et soit donc disponible dans une mesure élevée pour accentuer la vision floue causée par le défocus, et augmente la profondeur de champ naturelle, dans lequel, en raison des aberrations corrigées dans la zone d'implant optique, le traitement de signal latéral dans la rétine permet d'obtenir une vision monofocale optimale avec une acuité visuelle d'au moins 0,7 (70 %) dans une profondeur de champ d'au moins 2 dioptries, et
**qu'**après détermination d'une topographie théorique pour au moins l'une des deux surfaces de la zone d'implant optique, commandée par la topographie théorique déterminée, au moins l'une des deux surfaces d'une ébauche standard est usinée par enlèvement de copeaux ou par rayonnement laser ou est fabriquée par coulée directe au moyen de moules de coulée conçus de manière correspondante et que la topographie ainsi fabriquée est mesurée sur au moins une surface d'implant et comparée à la topographie théorique.

2. Procédé selon la revendication 1, **caractérisé en ce que**, pour la détermination de la topographie théorique, les données optiques d'au moins deux des composants de l'œil suivants sont mesurées biométriquement : cornée, chambre antérieure, cristallin naturel, position du cristallin naturel ou de la lentille artificielle déjà implantée en remplacement du cristallin naturel par rapport à la cornée, longueur axiale du globe oculaire.

3. Procédé selon la revendication 1 ou 2, **caractérisé en ce que** des données géométriques et/ou des indices de réfraction des composants de l'œil sont utilisés pour la détermination de la topographie théorique.

4. Procédé selon l'une des revendications 1 à 3, **caractérisé en ce que** le siège de la lentille intraoculaire à implanter dans l'œil est déterminé à partir des données de l'œil déterminées biométriquement.

5. Procédé selon l'une des revendications 1 à 4, **caractérisé en ce que** les données mesurées de la topographie produite par usinage mécanique par enlèvement de copeaux ou au laser à la surface de la lentille et/ou les données de la topographie théorique sont converties dans un format uniforme pour la comparaison.
